# DEMANDE DE BREVET EUROPEEN

(11) **EP 2 018 888 A1**
(43) Date de publication de la demande: **28.01.2009**
(21) Numéro de dépôt: 08160634.5
(22) Date de dépôt: 17.07.2008
(51) Int. Cl.: A61Q 5/00, A61Q 19/00, A61K 8/99, A61P 17/00, A61P 17/08, A61K 35/08, A61K 35/74

(54) **Utilisation d'un extrait bactérien cultivé sur une eau thermale pour le traitement des peaux sèches**

(30) Priorité: 17.07.2007 FR 0756531
(71) Demandeur: L'Oréal, 75008 Paris (FR)
(72) Inventeur: Gueniche, Audrey, 92500, Rueil Malmaison (FR); Breton, Lionel, 78000 Versailles (FR)
(74) Mandataire: Allab, Myriam

(57) **Abrégé**

La présente invention concerne l'utilisation d'au moins un extrait de bactérie filamenteuse non photosynthétique et non fructifiante cultivée sur un milieu comprenant au moins une eau minérale et/ou thermale non sulfureuse pour prévenir et/ou traiter la sécheresse des matières kératiniques, en particulier la sécheresse cutanée et notamment à traiter les désordres liés à une peau sèche et/ou hypo-séborrhéique.

## Description

La présente invention concerne l'utilisation d'au moins un extrait de bactérie filamenteuse non photosynthétique et non fructifiante cultivée sur un milieu comprenant au moins une eau minérale et/ou thermale non sulfureuse pour prévenir et/ou traiter la sécheresse des matières kératiniques, en particulier la sécheresse cutanée et notamment à traiter les désordres liés à une peau sèche et/ou hypo-séborrhéique.

On rappelle que la peau est constituée de trois couches superposées, de la surface vers la profondeur du corps : l'épiderme, le derme et l'hypoderme et comporte en outre des structures annexes telles que notamment les glandes sébacées.

La peau exerce essentiellement une fonction de barrière vis-à-vis du milieu extérieur qui résulte d'une organisation complexe et de nombreux facteurs. Cette fonction repose en particulier sur la qualité de l'épiderme qui dépend notamment du taux d'hydrophobicité de surface du *stratum corneum* et de l'équilibre entre la prolifération et la différenciation des kératinocytes épidermiques.

La rupture de l'équilibre cutané peut se manifester de différentes façons. Elle peut notamment conduire au déclenchement de processus inflammatoires, d'un dérèglement de la fonction sébacée, d'hyperkératinisation, ainsi qu'à une augmentation de la perte insensible en eau et plus généralement à une sécheresse cutanée. Ces événements affectent de façon négative le confort et/ou l'esthétique cutanée. Ils sont en outre susceptibles d'affecter l'état sanitaire de l'épiderme.

Ainsi, l'altération de la fonction barrière, va favoriser la pénétration anormale d'agents pathogènes dans la couche cornée et induire une libération accrue de substances pro-inflammatoires à l'origine de désordres inflammatoires cutanés qui provoquent des démangeaisons et tiraillements, deux symptômes caractéristiques d'une peau sèche.

Une première alternative de traitement des peaux sèches caractérisées par une déficience en lipides constitutifs de la barrière et/ou du film hydrolipidique, vise à administrer par voie topique des produits destinés à restaurer la barrière cutanée. Ces produits sont généralement des agents humidifiants, capables de fixer l'eau, des agents filmogènes destinés à retenir l'eau, des agents capables de reconstruire la barrière cutanée tels que les lipides exogènes constitutifs du ciment intercellulaire et du sébum tels que le squalène, les céramides, les acides gras ou encore des actifs à l'image de la vitamine C capables de stimuler la synthèse endogène des lipides épidermiques.

Comme précisé précédemment, la peau sèche peut être également la conséquence et/ou être associée à une insuffisance endogène de production de sébum par les glandes sébacées.

Conjointement à la sueur, le sébum constitue un hydratant naturel de l'épiderme et permet d'en accroître la souplesse et la résistance. Il est composé pour l'essentiel d'un mélange plus ou moins complexe de lipides. Classiquement, la glande sébacée produit du squalène, des triglycérides, des cires aliphatiques, des cires de cholestérol et, éventuellement, du cholestérol libre. C'est l'action des lipases bactériennes qui convertit une part variable des triglycérides formés en acides gras libres.

Classiquement, un taux de sébum inférieur à 100 µg/cm², mesuré au niveau de la zone T du visage, par la méthode décrite dans FR 2 368 708, peut être considéré comme caractéristique d'une peau sèche hypo-séborrhéique.

Un exemple de peau sèche hypo-séborrhéique, ou de peau le devenant est observé au cours du vieillissement cutané. Ainsi, il est très souvent constaté chez les sujets âgés et notamment de plus de 50 ans, la manifestation d'une xérose liée à une déficience en sébum. Par ailleurs, l'insuffisance de production de sébum peut être induite, par certains traitements pharmaceutiques comme ceux impliquant des corticoïdes.

La présente invention vise pour sa part à proposer l'utilisation de nouveaux composés dont l'efficacité a été constatée pour la prévention et/ou le traitement d'une manière générale des désordres associés à la sécheresse des matières kératiniques.

De manière inattendue, les inventeurs ont constaté que l'application topique d'un extrait de bactéries filamenteuses non fructifiantes cultivées sur un milieu enrichi en eau de la Roche Posay s'avérait tout particulièrement efficace, en particulier chez l'adulte, pour le traitement des peaux sèches. L'extrait de bactéries filamenteuses non fructifiantes cultivés sur un milieu enrichi en eau de la Roche Posay manifeste avantageusement une activité bénéfique au niveau de la barrière cutanée et limite la déshydratation de la peau.

Ainsi, la présente invention se rapporte à l'utilisation cosmétique d'au moins un extrait de bactérie filamenteuse non photosynthétique et non fructifiante cultivée sur un milieu comprenant au moins une eau thermale et/ou minérale non sulfureuse comme agent pour traiter les signes associés à la sécheresse des matières kératiniques.

Au sens de la présente invention, "matière kératinique" désigne la peau, le cuir chevelu, les muqueuses et semi-muqueuses, les ongles, et les fibres kératiniques d'origine humaine ou animale.

### Extrait bactérien

L'extrait bactérien utilisé selon l'invention est préparé suivant un procédé comprenant la culture d'au moins une bactérie filamenteuse non photosynthétique et non fructifiante sur un milieu comprenant au moins une eau minérale et/ou thermale non sulfureuse.

Les bactéries utilisées sont des bactéries filamenteuses non photosynthétiques qui comprennent notamment les bactéries appartenant à l'ordre des Beggiatoales, et plus particulièrement les bactéries appartenant aux genres Beggiatoa, Vitreoscilla, Flexithrix ou Leucothrix.

Pour la mise en oeuvre de l'invention, on préfère les bactéries appartenant au genre Vitreoscilla, en particulier pour les bactéries de l'espèce *Vitreoscilla filiformis.*

Ces bactéries dont plusieurs ont déjà été décrites ont généralement un habitat aquatique, et peuvent être trouvées notamment dans des eaux marines ou dans des eaux thermales. Parmi les bactéries utilisables, on peut citer par exemple :
*Vitreoscilla filiformis* (ATCC 15551)
*Vitreoscilla beggiatoïdes* (ATCC 43181)
*Beggiatoa alba* (ATCC 33555)
*Flexithrix dorotheae* (ATCC 23163)
*Leucothrix mucor* (ATCC 25107)
*Sphaerotilus natans* (ATCC 13338)

De préférence, la bactérie est celle correspondant à la souche déposée à l'ATCC sous le n°15551.

Par eau thermale, on entend une eau chaude ou froide qui est utilisée pour ses vertus thérapeutiques ou pour un usage balnéaire. On peut utiliser une eau thermale ou une eau minérale. En général, une eau minérale est propre à la consommation, ce qui n'est pas toujours le cas d'une eau thermale. Chacune de ces eaux contient, entre autre, des minéraux solubilisés et des oligoéléments. Ces eaux sont connues pour être employées à des fins de traitement spécifique selon les oligo-éléments et les minéraux particuliers qu'elles contiennent.

De préférence, on utilise une eau thermale et/ou minérale qui présente une minéralisation supérieure ou égale à 400 mg/l.
On entend, dans l'invention, par "minéralisation", la somme des concentrations en anions et en cations présents dans l'eau thermale ou minérale. Dans les eaux thermales ou minérales utiles selon l'invention, la minéralisation est généralement comprise entre 400 et 900 mg/l.

L'eau thermale et/ou minérale utilisée selon l'invention peut avoir une minéralisation d'au moins 700 mg/l et, en particulier, une concentration totale en carbonates et en bicarbonates d'au moins 150 mg/l et plus préférentiellement d'au moins 360 mg/l et notamment en carbonate et bicarbonate de sodium supérieure à 2 mg/l. La concentration en oxyde de silicium dans l'eau utilisée dans la composition selon l'invention peut être de préférence d'au moins 6 mg/l et plus préférentiellement d'au moins 9 mg/l.

L'eau thermale ou l'eau minérale utilisée selon l'invention peut être choisie parmi l'eau de Vittel, les eaux du bassin de Vichy, l'eau d'Uriage, l'eau de la Roche Posay, l'eau de la Bourboule, l'eau d'Enghien-les-bains, l'eau de Saint-Gervais-les bains, l'eau de Néris-les-bains, l'eau d'Allevard-les-bains, l'eau de Digne, l'eau des Maizières, l'eau de Neyrac-les-bains, l'eau de Lons le Saunier, les Eaux Bonnes, l'eau de Rochefort, l'eau de Saint Christau, l'eau des Fumades et l'eau de Tercis-les-bains.
Avantageusement, elle est choisie parmi les eaux non sulfureuses telles que l'eau de Vittel, les eaux du bassin de Vichy, l'eau de la Roche Posay, l'eau de la Bourboule, l'eau d'Enghien-les-bains, l'eau de Saint-Gervais-les bains, l'eau de Néris-les-bains, l'eau d'Allevard-les-bains, l'eau de Digne, l'eau des Maizières, l'eau de Neyrac-les-bains, l'eau de Lons le Saunier, l'eau de Rochefort, l'eau de Saint Christau et l'eau de Tercis-les-bains.

Parmi ces eaux, celles qui présentent une concentration totale en carbonates ou bicarbonates supérieure à 360 mg/l sont l'eau de Vittel, l'eau de la Bourboule, l'eau des Fumades, l'eau d'Enghien-les-bains, l'eau de la Roche-Posay, l'eau du bassin de Vichy, l'eau d'Uriage.

Parmi ces eaux celles qui présentent une concentration en carbonates ou bicarbonates comprise entre 150 mg/l et 360 mg/l sont l'eau de Digne, l'eau de Maizières, l'eau de Rochefort, l'eau de Saint-Gervais-les-bains.

Parmi ces eaux, celles qui contiennent au moins 2 mg/l de carbonate ou bicarbonate de sodium sont l'eau de la Roche Posay, l'eau de Vittel, les eaux du bassin de Vichy, l'eau d'Uriage.

Les eaux contenant au moins 9 mg/l d'oxyde de silicium sont l'eau de la Roche Posay, l'eau de Vittel, les eaux du bassin de Vichy, l'eau d'Uriage.

Les eaux thermales ou minérales convenant particulièrement à la mise en oeuvre de l'invention ont une concentration en ions calcium supérieure ou égale à 100 mg/l, voire à 140 mg/l.
Selon un mode de réalisation avantageux, l'eau thermale ou minérale a une concentration en ions hydrogénocarbonates supérieure ou égale à 300 mg/l. Les hydrogénocarbonates, aussi appelés bicarbonates, sont notamment présents à une concentration supérieure ou égale à 350 mg/l.

Selon un mode de réalisation avantageux, les bactéries sont cultivées sur un milieu comprenant au moins une eau thermale. Celle-ci peut en particulier être choisie parmi l'eau de Vittel, les eaux du bassin de Vichy, l'eau d'Uriage, l'eau de la Roche-Posay, l'eau de la Bourboule, l'eau des Fumades, l'eau d'Enghien-les-bains, les Eaux Bonnes, et notamment parmi l'eau de Vittel, les eaux du bassin de Vichy, l'eau de la Roche-Posay, l'eau de la Bourboule et l'eau d'Enghien-les-bains.

Les eaux permettant d'obtenir un résultat particulièrement avantageux selon l'invention sont notamment choisies parmi l'eau de la Roche Posay et l'eau de Vittel, ou une eau de composition similaire.
L'eau de la Roche Posay est extraite de la source du même nom, il s'agit d'une eau bicarbonatée calcique, silicatée et séléniée. Elle comprend généralement environ 387 mg/l d'ions bicarbonates, environ 140 mg/l d'ions calcium et au moins 4 mg/l de sulfates.
L'eau de Vittel est riche en calcium et sels minéraux (841 mg/l) et contient notamment 202 mg/l de calcium, 402 mg/l de bicarbonates et 336 mg/l de sulfates.

On peut en particulier effectuer une culture dans le milieu suivant :

| **Composition** | **Concentration** |
|---|---|
| Extrait autolytique de levure | 0,5 à 5 g/l |
| Peptone végétale | 0,5 à 5 g/l |
| Glucose anhydre | 0,5 à 7 g/l |
| Micro-éléments de Heller | 0,5 à 5 ml/l |
| CaCl₂, 10 H₂O | 0,010 à 0,200 g/l |

On complète à 1000 ml par de l'eau minérale et/ou thermale éventuellement complétée d'eau distillée ou osmosée.

Parmi les peptones utilisables, on peut citer par exemple la peptone papaïnique de soja.
Ce milieu se distingue des milieux généralement utilisés par l'absence de catalase et de sulfure.
Les micro-éléments de Heller ont été décrits par Heller, Ann Sci. Nat. Biol. Veg. 14 : 1-223 (1953). Il s'agit de mélanges de divers éléments minéraux qui ont été recommandés par Heller, non pas pour la culture des bactéries, mais pour la nutrition des tissus végétaux cultivés *in vitro.*

La culture peut être effectuée à la température appropriée convenant pour l'espèce bactérienne cultivée. Généralement cette température est comprise entre 18 et 40 °C suivant les souches. Le pH du milieu de culture est de préférence compris entre 5,5 et 8.

La composition des micro-éléments de Heller, pour 1l d'eau, est la suivante:

| | |
|---|---|
| ZnSO₄,7 H₂O | 1 g |
| MnSO₄,H₂O | 0,076 g |
| CuSO₄,5H₂O | 0,003 g |
| Kl | 0,010 g |
| H₃BO₃ | 1 g |
| AlCl₃,6H₂O | 0,050 g |
| NiCl₂,6H₂O | 0,030 g |

Lesdites eaux thermales ou minérales peuvent représenter tout ou partie de la phase aqueuse du milieu de culture. Elles peuvent ainsi se trouver en mélange en toute proportion avec l'eau, en particulier distillée ou osmosée, présente dans le milieu de culture. Le mélange (i) d'eau thermale et (ii) d'eau osmosée ou distillée pourra être dans un ratio compris entre 0,1% et 100%, notamment de 0,1 à 50, en particulier de 0,1 à 25.
En particulier, un extrait de bactérie filamenteuse convenant à l'utilisation selon l'invention est susceptible d'être obtenu par culture de ladite bactérie sur un milieu de culture qui contient un mélange (i) d'eau osmosée ou distillée et (ii) d'eau thermale, dans un ratio (i)/(ii) compris entre 1 et 100, notamment de 1 à 50, en particulier de 1 à 25.
On utilisera en particulier l'eau thermale de La Roche Posay, telle que définie dans ce qui précède.

Après mélange de tous les éléments du milieu, on procède avantageusement à une stérilisation du milieu de culture contenant l'eau thermale et/ou minérale; cette étape est effectuée par des méthodes connues de l'homme du métier telles que la stérilisation par filtration ou par la chaleur.

Le milieu de culture est ensuite ensemencé par les bactéries.

Les milieux convenant le mieux à la culture des bactéries sont tels que l'eau thermale ou minérale représente de préférence au moins 0,1% de la quantité d'eau introduite pour la préparation du milieu, notamment de 0,1 à 99,9%. De bons résultats sont obtenus avec des concentrations d'eau thermale d'environ 1 ou 2%, notamment d'environ 1,33% par rapport à l'eau osmosée et/ou distillée, par exemple de 0,5 à 20%, voire de 0,5 à 50%, mais ces concentrations peuvent être augmentées sans inconvénient.

De façon connue, le procédé de préparation de l'extrait bactérien comprend au moins une étape dans laquelle on récupère les bactéries à la fin de la culture, en particulier en les séparant du milieu de culture.

Après culture des bactéries, on peut isoler la biomasse par diverses méthodes connues, par exemple par filtration, par coagulation avec un alcool (éthanol, isopropanol, isobutanol), par séchage sur cylindre à précouche (amidon, diatomées...) raclée, ou par lyophilisation. Une concentration préalable, par exemple à 80°C sous pression réduite, améliore cette séparation.
La biomasse peut être utilisée vivante ou bien être traitée par différents procédés. On peut procéder à une opération de rupture des enveloppes, par exemple par l'action des ultrasons. On peut en outre préparer des extraits à l'aide d'un alcool tel que l'éthanol ou le propanol. On peut également préparer des extraits lipopolysaccharidiques selon les méthodes connues, voir par exemple Noris et Ribbons, Methods in Microbiology, Vol. 5B, Academic Press (1971). La méthode généralement utilisée est la méthode bien connue dite de Westphal (ou une méthode apparentée), qui consiste à faire l'extraction avec des mélanges phénol-eau à 65°C. On soumet ensuite l'extrait à une dialyse pour éliminer le phénol.

L'extrait bactérien utile selon l'invention peut encore résulter de la mise en oeuvre du procédé suivant : (i) on cultive au moins une bactérie appartenant à l'ordre des Beggiatoales sur un milieu comprenant un ose comme source principale de carbone et au moins une eau minérale ou thermale, puis (ii) après fermentation, on sépare les bactéries du milieu de culture, pour récupérer ladite masse des bactéries.

Les bactéries récupérées à l'issue de l'étape de fermentation peuvent notamment être soumises à un traitement de stabilisation et/ou d'extraction. C'est l'extrait de bactéries filamenteuses ainsi obtenu qui sera généralement utilisé dans ou pour la préparation de compositions cosmétiques ou dermatologiques. De manière connue en soi, l'extrait pourra ainsi être stérilisé en particulier par filtration ou par autoclavage.

Par extrait de bactéries filamenteuses non photosynthétiques, on entend aussi bien le surnageant de culture desdites bactéries, la biomasse obtenue après culture desdites bactéries ou encore les extraits de la biomasse obtenus par traitement de cette biomasse. Pour préparer l'extrait selon l'invention, on peut cultiver lesdites bactéries selon le procédé selon l'invention, puis les séparer de la biomasse obtenue, par exemple par filtration, centrifugation, coagulation et/ou lyophilisation.

Ainsi, après culture, les bactéries sont concentrées par centrifugation. La biomasse obtenue est autoclavée. Cette biomasse peut être lyophilisée pour constituer ce que l'on appelle l'extrait lyophilisé. Toute méthode de lyophilisation connue de l'homme du métier est utilisable pour préparer cet extrait.
La fraction surnageante de cette biomasse peut également être filtrée dans un récipient stérile pour éliminer les particules en suspension. Cette fraction surnageante peut également être transvasée stérilement dans un récipient stérile. Selon un mode de réalisation particulier de l'invention, la fraction surnageante ainsi obtenue est utilisée à titre d'actif cosmétique ou dermatologique.

L'extrait bactérien utile selon l'invention peut être formulé dans un support approprié à raison d'au moins 20% en poids par rapport au poids total de la composition, en particulier à raison de 0,001 à 20% en poids par rapport au poids total de la composition et plus particulièrement à raison de 0,01 à 10% en poids par rapport au poids total de la composition.
Pour certaines applications ou formulations spécifiques, il peut être avantageux d'utiliser des concentrations pondérales élevées en extrait bactérien, par exemple, comprises entre 15 à 20%.

L'extrait bactérien cultivé sur eau thermale utile selon l'invention peut-être utilisé sous une forme poudre lyophilisée, par exemple, qui peut atteindre des % de l'ordre de 0,001% à 20%, en poids d'extrait sec de bactéries filamenteuses non fructifiantes non photosythétiques par rapport au poids de la composition. Dans des formes d'application particulières de type balnéothérapie, on peut également envisager des applications en des proportions supérieures.

L'extrait bactérien cultivé sur un milieu enrichi en eau thermale peut encore être utilisé sous forme de fractions de composants cellulaires ou sous la forme de métabolites. Le(s) microorganisme(s), métabolite(s) ou fraction(s) peu(ven)t également être introduit(s) sous la forme d'une poudre lyophilisée, d'un surnageant de culture et/ou le cas échéant sous une forme concentrée.

Pour certaines applications, la biomasse vivante peut être utilisée telle qu'elle par exemple sous forme de masques ou de cataplasme pour produire un effet immédiat.

Au sens de l'invention, le terme "métabolite" désigne toute substance issue du métabolisme des microorganismes considérés selon l'invention et dotée d'une efficacité pour le traitement des peaux sèches.

De manière inattendue, la Demanderesse a constaté que les extraits bactériens cultivés sur eau thermale pouvaient s'avérer efficaces pour le traitement des matières kératiniques sèches.

En effet, la Demanderesse a pu mettre en évidence que l'extrait de la bactérie *Vitreoscilla filiformis* cultivée sur l'eau thermale de la Roche Posay a une efficacité de traitement des peaux sèches accrue par rapport à l'extrait de la même bactérie cultivée sur milieu classique, c'est-à-dire sans eau minérale ou thermale.
La différence essentielle entre ces deux extraits réside dans les protocoles de préparation du milieu de culture où il y a substitution , au moins en partie, de l'eau osmosée par l'eau de La Roche Posay. Sans que la Demanderesse ne soit tenue à cette hypothèse, cela conduirait notamment à une modification du métabolisme des bactéries causée par un enrichissement du milieu de culture en éléments minéraux, particulièrement en sélénium, strontium et zinc.

Il est également intéressant de noter que l'introduction de cette biomasse dans un support formulatoire n'entraîne pas de risque de surexposition à ces éléments, Se et Zn sont des éléments essentiels à l'organisme et le Sr est largement répandu dans l'alimentation.
Le tableau ci-dessous fournit les concentrations de ces éléments chimiques dans l'extrait bactérien selon l'invention préparé selon le protocole de l'exemple 1 (extrait lyophilisé).

| | |
|---|---|
| Se (mg/kg) | 6 |
| Sr (mg/kg) | 10 |
| Zn (mg/kg) | 216 |

Ainsi, l'application de cet extrait enrichi entraîne des expositions topiques en sels minéraux par jour de l'ordre de :

| | |
|---|---|
| Se (µg/J) | 0,008 |
| Sr (µg/J) | 0,0032 |
| Zn (µg/J) | 0,094 |

Rappelons ici que l'utilisation des ions pour l'amélioration de l'état cutané est très ancienne. Ainsi, les cures thermales à visée dermatologique sur les bords de la Mer Morte -étendue d'eau la plus saline du monde- remontent à l'Antiquité (Abels DJ et coll, Clinics in Dermatol 14 : 653-658,1996).

Ainsi l'invention se rapporte à l'utilisation cosmétique d'au moins un extrait de bactérie filamenteuse non photosynthétique et non fructifiante cultivée sur un milieu comprenant au moins une eau thermale et/ou minérale non sulfureuse comme agent pour traiter les signes associés à la sécheresse des matières kératiniques.

Par traiter, sauf indication contraire, on entend toute action visant à améliorer le confort, le bien-être d'un individu, ce terme couvre donc aussi bien prévenir, atténuer, réduire, soulager que curer.

En particulier, ledit extrait de bactérie est utile comme agent :
- pour traiter les états de sècheresse cutanée, les peaux d'aspect rugueux à la vue et/ou au toucher ; les états squameux ; notamment les états pelliculaires ;
- pour traiter les peaux sèches ;
- pour traiter les démangeaisons et/ou tiraillements associés aux peaux sèches ;
- pour traiter les désordres cutanés liés à un défaut d'excrétion et/ou de sécrétion de sébum ;
- pour restaurer physiologiquement un état d'hydratation convenable au *stratum corneum* ;
- pour traiter les peaux sèches hypo-séborrhéiques ;
- pour stimuler la sébogénèse ;
- pour traiter les fibres kératiniques sèches ;
Dans le cas des fibres kératiniques humaines ou animales, à l'image des cheveux, poils et/ou cils, l'extrait bactérien utilisé selon l'invention s'avère particulièrement avantageux pour prévenir et/ou traiter la manifestation des signes de fragilité, comme par exemple la sécheresse qui se traduit, généralement, par un aspect cassant de la fibre. L'extrait permet ainsi de conférer un aspect lustré aux fibres kératiniques, en particulier à la chevelure humaine et au pelage animal.
- pour traiter les désordres fonctionnels de l'unité pilo-sébacée ;
- pour prévenir et/ou réduire les rides liées à une sécheresse cutanée ;
- d'améliorer le confort des peaux et cuirs chevelus secs ;
- lutter contre l'aspect terne et/ou atone de la peau et/ou des cheveux, conséquences de leur dessèchement.

La sécheresse cutanée est souvent associée à une baisse du taux d'hydratation cutanée, évalué par cornéométrie, et à une altération de la fonction barrière, mesurée par la perte insensible en eau.
La peau sèche se manifeste essentiellement par une sensation de tiraillements et/ou de tension. Celle-ci est aussi rugueuse au toucher et apparaît couverte de squames. Lorsque la peau est légèrement sèche, ces squames sont abondantes mais peu visibles à l'oeil nu. Elles sont de moins en moins nombreuses mais de plus en plus visibles à l'oeil nu lorsque ce désordre s'aggrave.

L'origine de la sécheresse cutanée peut être de type constitutionnel ou acquis.

Dans le cas de peau sèche acquise, l'intervention de paramètres extérieurs tels que l'exposition aux agents chimiques, à des conditions climatiques difficiles, aux rayons solaires ou bien encore certains traitements thérapeutiques (rétinoïdes, par exemple) est déterminante. Sous ces influences extérieures, la peau peut devenir alors momentanément et localement sèche. Cela peut concerner tout type de peau, normal et même gras.

Dans le cas de la peau sèche constitutionnelle, on peut distinguer deux catégories : les peaux pathologiques et les peaux non pathologiques.

Les peaux sèches constitutionnelles pathologiques sont essentiellement représentées par la dermatite atopique et les ichthyoses, les hyperkératoses. Elles sont quasiment indépendantes des conditions extérieures.
La dermatite atopique est décrite comme associée à un déficit dans le métabolisme des lipides du *stratum corneum* et notamment des céramides. Cette pathologie se présente sous la forme d'une xérose plus ou moins chronique concernant une grande étendue du corps, associée à des poussées inflammatoires et prurigineuses par plaques.

Les ichthyoses sont des pathologies caractérisées par un déficit génétique affectant le processus de kératinisation à différents stades. Elles se manifestent par une desquamation importante par plaques.

Les peaux sèches constitutionnelles non pathologiques sont des peaux sèches dont la sévérité peut dépendre des facteurs extérieurs déjà évoqués. Rentrent dans cette catégorie de peau, la peau sénile (caractérisée par une diminution générale du métabolisme cutané avec l'âge), la peau fragile (très sensible aux facteurs extérieurs et souvent accompagnée d'érythème et de rosacée) et la xérose vulgaire (d'origine génétique probable et se manifestant en priorité sur le visage, les membres et le dos des mains).

L'utilisation selon l'invention s'avère ainsi tout particulièrement efficace pour prévenir et/ou traiter les peaux sèches et plus particulièrement les peaux sèches acquises et/ou les peaux sèches constitutionnelles.

L'extrait bactérien selon l'invention est donc utilisé pour la préparation de compositions destinées au traitement prophylactique ou thérapeutique de la dermatite atopique (dans les phases de rémission comme traitement d'entretien), des xéroses atopiques, des dermites séborrheiques et des hyperkératoses.

L'invention se rapporte encore à l'extrait de bactérie filamenteuse non photosynthétique et non fructifiante cultivée sur un milieu comprenant au moins une eau thermale et/ou minérale non sulfureuse pour son utilisation pour le traitement prophylactique ou thérapeutique de la dermatite atopique (dans les phases de rémission comme traitement d'entretien), des xéroses atopiques, des dermites séborrheiques et des hyperkératoses.

Les extraits bactériens utilisés selon l'invention peuvent être avantageusement associés à d'autres actifs.
A titre d'actifs utilisables, on peut citer les vitamines B3, B5, B6, B8, C, E ou PP, les caroténoides, les curcuminoides et la niacine.

L'utilisation selon l'invention se fait par toute voie d'administration adaptée à l'effet recherché, notamment par voie orale ou topique, avantageusement par voie topique sur la peau.

Par voie topique, on entend une administration des extraits selon l'invention ou des compositions qui le comprennent par application sur la peau telle que définie ci-dessus. Sauf indication contraire, dans le cadre de l'invention, par peau, on entend toute surface cutanée du corps incluant la peau et élargie au cuir chevelu et aux muqueuses et semi-muqueuses et par phanères, on entend les cils, poils, cheveux et ongles.

Les compositions topiques selon l'invention qui sont destinées au traitement de la peau, des muqueuses et semi-muqueuses et du cuir chevelu, peuvent se présenter sous forme d'onguent, de crèmes, de laits, de pommades, de poudres, de tampons imbibés, de solutions, de gels, de sprays, de lotions, ou de suspensions. Elles peuvent également se présenter sous forme de microsphères ou nanosphères ou de vésicules lipidiques ou polymériques ou de patchs polymériques et d'hydrogels permettant une libération contrôlée. Ces compositions par voie topique peuvent se présenter soit sous forme anhydre, soit sous forme aqueuse selon l'indication dermocosmétique.

Les compositions sont notamment destinées à l'hygiène capillaire. Elles peuvent se présenter notamment sous forme d'une crème, d'un lait, d'une lotion, d'un gel, de microsphères ou de nanosphères ou vésicules lipidiques ou polymèriques, d'un savon ou d'un shampoing.

La présente invention se rapporte également à une composition associant au moins un extrait bactérien utile selon l'invention en association avec d'autres actifs.
Il est avantageux d'introduire dans la composition selon l'invention au moins un composé choisi parmi : les agents desquamants ; les agents hydratants ; les agents anti-inflammatoires ou apaisants ; les agents stimulant la prolifération et/ou la différenciation des kératinocytes ; les agents anti-pelliculaires ; et les agents anti-microbiens.

En effet, la stimulation de la séborrhée par l'extrait bactérien selon l'invention peut, chez certaines personnes, fournir un terrain de prolifération pour la microflore résidente de l'ostium folliculaire (*Propionibacterium acnes* en particulier), provoquant ainsi une hydrolyse importante des triglycérides du sébum en acides gras libres et la réduction des insaturations des acides gras poly-insaturés (acide linoléique en particulier). Ces deux phénomènes peuvent concourir à une kératinisation de l'infundibulum et à la formation d'un micro-comédon. Celui-ci peut dégénérer en comédon, bouchant et dilatant le pore de façon inesthétique. A un stade plus avancé, ce bouchon peut diverger vers une lésion acnéique inflammatoire.
L'ajout d'agents desquamants ou stimulant la prolifération ou la différenciation des kératinocytes à la composition selon l'invention permettent d'éviter la formation de ces comédons. De même, des agents anti-microbiens, anti-bactériens ou bactériostatiques permettent, en modérant la prolifération de la microflore résidente, d'obtenir le même effet.

En outre, les agents hydratants peuvent compléter l'effet obtenu à l'aide des extraits bactériens selon l'invention, et les agents apaisants sont utiles pour améliorer le confort des peaux sèches oligoséborrhéiques.

Enfin, l'utilisation d'agents anti-pelliculaires est utile dans le cas où la composition selon l'invention est destinée au traitement des cuirs chevelus secs.

### Agent desquamant

Par "agent desquamant", on entend tout composé capable d'agir :
- soit directement sur la desquamation en favorisant l'exfoliation, tel que les β-hydroxyacides, en particulier l'acide salicylique et ses dérivés (dont l'acide n-octanoyl 5-salicylique) ; les α-hydroxyacides, tels que les acides glycolique, citrique, lactique, tartrique, malique ou mandélique ; l'urée ; l'acide gentisique ; les oligofucoses ; l'acide cinnamique ; l'extrait de Saphora japonica ; le resvératrol et certains dérivés d'acide jasmonique ;
- soit sur les enzymes impliquées dans la desquamation ou la dégradation des cornéodesmosomes, les glycosidases, la stratum corneum chymotryptic enzym (SCCE) voire d'autres protéases (trypsine, chymotrypsine-like). On peut citer les agents chélatant des sels minéraux : l'EDTA ; l'acide N-acyl-N,N',N' éthylène diaminetriacétique ; les composés aminosulfoniques et en particulier l'acide (N-2 hydroxyéthylpiperazine-N-2-éthane) sulfonique (HEPES) ; les dérivés de l'acide 2-oxothiazolidine-4-carboxylique (procystéine) ; les dérivés d'acides alpha aminés de type glycine (tels que décrits dans EP-0 852 949, ainsi que le méthyl glycine diacétate de sodium commercialisé par BASF sous la dénomination commerciale TRILON M) ; le miel ; les dérivés de sucre tels que l'O-octanoyl-6-D-maltose et la N-acétyl glucosamine.

### Agent hydratant

Par "agent hydratant", on entend :
- soit un composé agissant sur la fonction barrière, en vue de maintenir l'hydratation du stratum corneum, ou un composé occlusif. On peut citer les céramides, les composés à base sphingoïde, les lécithines, les glycosphingolipides, les phospholipides, le cholestérol et ses dérivés, les phytostérols (stigmastérol, β-sitostérol, campestérol), les acides gras essentiels, le 1-2 diacylglycérol, la 4-chromanone, les triterpènes pentacycliques tels que l'acide ursolique, la vaseline et la lanoline ;
- soit un composé augmentant directement la teneur en eau du stratum corneum, tel que le thréalose et ses dérivés, l'acide hyaluronique et ses dérivés, le glycérol, le pentanediol, le pidolate de sodium, la sérine, le xylitol, le lactate de sodium, le polyacrylate de glycérol, l'ectoïne et ses dérivés, le chitosane, les oligo- et polysaccharides comme le produit commercialisé sous la référence Pentavitin, le miel, les alginates (notamment le produit Sobalg PH 154 commercialisé par la société Grindsted), les carbonates cycliques, l'acide N-lauroyl pyrrolidone carboxylique ou ses sels, notamment le sel de sodium commercialisé sous la référence Nalidone, et la N-α-benzoyl-L-arginine ;
- soit un composé activant les glandes sébacées tel que les dérivés stéroïdiens (dont la DHEA, ses dérivés 7-oxydés et/ou 17-alkylés et les sapogénines), le dihydrojasmonate de méthyle, et la vitamine D et ses dérivés.
Ces composés peuvent représenter de 0,001% à 30%, et de préférence de 0,01 à 20%, du poids total de la composition selon l'invention.

### Agent anti-glycation

Par "agent anti-glycation", on entend un composé prévenant et/ou diminuant la glycation des protéines de la peau, en particulier des protéines du derme telles que le collagène.
Des exemples d'agents anti-glycation sont les extraits végétaux de la famille des Ericaceae, tels qu'un extrait de myrtille (*Vaccinium angusfifollium*) ; l'ergothionéine et ses dérivés ; et les hydroxystilbènes et leurs dérivés, tels que le resvératrol et le 3,3', 5,5'-tétrahydroxystilbène. Ces agents anti-glycation sont décrits dans les demandes FR 99/16166, FR 00/08158, FR 99/09267 et FR 99/16168, respectivement. Le resvératrol est particulièrement préféré pour une utilisation dans cette invention.

La composition selon l'invention comprenant un agent anti-glycation tel que défini ci-dessus peut avantageusement être utilisée pour prévenir ou traiter les signes du vieillissement cutané, en particulier pour prévenir ou traiter la perte de tonicité et/ou d'élasticité de la peau.

### Agents anti-inflammatoires et/ou apaisants

- un antagoniste de cytokines inflammatoires ;
- un anti-inflammatoire stéroïdien (Hydrocortisone, betaméthasone, dexaméthasone etc..) ;
- un anti-inflammatoire non stéroïdien comme l'aspirine ou le paracetamol ;
- l'acide bêta-glycyrrhétinique, les extraits en contenant comme par exemple l'extrait de Glycyrrhiza Glabra (réglisse) et les complexes en contenant comme le complexe allantoïne/acide glycyrrhétinique ;
- les planctons, lyophilisés ou non, leurs extraits et leurs complexes ;
- l'escine et les extraits végétaux en contenant comme l'extrait de marron d'inde ;
- les dérivés de xanthine comme le chlorhydrate de di-éthylaminoéthyl théophylline et la caféine ;
- les eaux et extraits (par exemple aqueux, hydroalcooliques ou hydroglycoliques) de fleurs et de plantes, comme l'eau de bleuet, l'eau de camomille, l'eau de menthe, l'eau de tilleul, l'eau de rose, les extraits de Rosacées (ex : *Rosa gallica*), les extraits de pivoine, les extraits d'aubépine, les extraits de millefeuille, les extraits de mauve, les extraits de souci, les extraits de melilot, les extraits de sauge, l'eau de sureau, les extraits de ginkgo biloba, les extraits d'arnica, les extraits d'origan, les extraits de thé vert, les extraits de fleurs de nénuphar, les extraits d'Iris, les extraits d'écorce de bouleau, les extraits d'Aloe vera ;
- l'acide asiatique et les extraits végétaux en contenant comme la Centella Asiatica ;
- les extraits de fruits, comme l'extrait d'ananas, l'extrait de papaye ; de goyave ;
- les algues notamment du type *Laminaria* (par exemple rouges ou brunes) ;
- les pyrrolidone carboxylates et notamment de zinc (Zn-PCA) ou de cuivre (Cu-PCA) ;
- les huiles d'origine végétale, comme l'huile de graine de canola et l'huile de karité ;
- les huiles essentielles, par exemple de coriandre, de mélisse, de lavande, de menthe, de camomille et leurs mélanges ;
- l'acide acexamique et l'acide transexamique (acide trans-4, aminométhylcyclohexane carboxylique) ;
- l'acide ursolique et les extraits en contenant comme l'extrait de feuille de romarin ;
- les polysaccharides contenant du fucose, comme le FUCOGEL 1000, vendu par Solabia (solution aqueuse comprenant 1% de matière sèche de polysaccharide comprenant du fucose, du galactose et de l'acide galacturonique) ;
- les électrolytes et en particulier un mélange aqueux comprenant de 30 à 35 % du chlorure de magnésium, de 20 à 28 % de chlorure de potassium, de 3 à 10 % de chlorure de sodium, de 0,2 à 1 % de chlorure de calcium, de 0,1 à 0,6 % de bromure de magnésium et de 0,1 à 0,5 % d'insolubles, le dit mélange étant ici appelé "mélange des sels de la mer Morte" (« Dead Sea Bath Salts ») car il correspond aux principaux sels contenus dans la mer Morte ;
- les galactolipides par exemple issus d'avoine, tels que par exemple le digalactosyl diglycéride ou le monogalactosyl diglycéride ;
- les acides aminés, leurs dérivés et leurs sels, tels que le sel de sodium d'aminoacides greffés sur des chaînes cocoyle, commercialisé sous forme d'un mélange sous la dénomination SEPICALM S par la société SEPPIC, le capryloylglycine commercialisé sous la dénomination LIPACIDE C8G par la société SEPPIC et le mélange de capryloylglycine, de cannelle et de sarcosine commercialisé sous la dénomination SEPICONTROL A5 par la société SEPPIC ;
- les antagonistes de TNF-alpha tels que la lisophylline, l'A802715, la sulfasalazine, le CDP-571 (anticorps anti-TNF-alpha), le MDL-201112 ;
- les antagonistes de substance P tels que le sendide, le spantide II, et les peptides décrits dans la demande EP-A-680749, les extraits de bactérie filamenteuse décrits dans la demande EP-A-761204 ;
- les antagonistes de CGRP, tels que le CGRP 8-37, les anticorps anti-CGRP, ou des extraits végétaux à activité antagoniste du CGRP (ex : *Iris pallida*).
- les sels divalents de strontium, de zinc, de manganèse, de magnésium, de calcium, tels que ceux décrits dans les documents WO-A-96/19184, WO-A-96/19182 et WO-A-96/19228 ;
et leurs mélanges.

Par 'antagonistes de cytokines inflammatoires' selon l'invention, on entend un composé susceptible d'inhiber la synthèse et/ou la libération d'une ou plusieurs cytokines inflammatoires. Entrent également dans la définition d'un antagoniste des cytokines inflammatoires, les composés qui inhibent ou bloquent la fixation de ces cytokines sur leur(s) récepteur(s).
On peut citer par exemple les composés antagonistes de l'IL-1, de l'IL-8, du TNFα et du TNFβ, le tripeptide Lys-Pro-Val (KPV), et tous les dérivés de l'αMSH et peptidométiques apparentés caractérisés par une activité de type αMSH par liaison sur le récepteur ou par le contrôle de la libération d'IL1, d'IL8 ou encore de TNFα, tous les inhibiteurs de libération des cytokines (classe thérapeutique des CSAIDs pour : cytokine suppressive anti-inflammatory drugs), la famille des pyrimidine N-Oxyde substitués (EP99401719.2 (priorité FR9809509) et EP 99402771.2 (priorité FR9814211) inducteurs de lipoxine A4, les extraits d'algues capable de moduler la production des cytokines par les kératinocytes comme le Phycosaccharide® commercialisé par la société CODIF, la Phlorogine®, commercialisée par la société SECMA, les extraits *d'Aloe vera* ou de *Gingko biloba*; l'antagoniste naturel à l'IL-1 (IL-1 RA).

On peut également citer des peptides immunomodulateurs, tels que le polypeptide Gly-Gln-Pro-Arg et dérivés décrit dans la demande PCT/FR00/00031.

Les agents anti-inflammatoires sont de préférence présents dans les compositions conformes à l'invention à une concentration pouvant varier entre 0,00001 et 10 % en poids environ par rapport au poids total de la composition. Encore plus préférentiellement, la concentration en composé anti-inflammatoire peut varier entre 0.0005% et 2 % en poids par rapport au poids total de la composition.

### Agent stimulant la prolifération des fibroblastes ou des kératinocytes et/ou la différenciation des kératinocytes

Les agents stimulant la prolifération des fibroblastes utilisables dans la composition selon l'invention peuvent par exemple être choisis parmi les protéines ou polypeptides végétaux, extraits notamment du soja (par exemple un extrait de soja commercialisé par la société LSN sous la dénomination Eleseryl SH-VEG 8^{®} ou commercialisé par la société SILAB sous la dénomination commerciale Raffermine^{®}) ; et les hormones végétales telles que les giberrellines et les cytokinines
Les agents stimulant la prolifération des kératinocytes, utilisables dans la composition selon l'invention, comprennent notamment les rétinoïdes tels que le rétinol et ses esters, dont le palmitate de rétinyle ; l'adénosine ; le phloroglucinol ; les extraits de tourteaux de noix commercialisés par la société GATTEFOSSE ; et les extraits de Solanum tuberosum commercialisés par la société SEDERMA.
Les agents stimulant la différenciation des kératinocytes comprennent par exemple les minéraux tels que le calcium ; un extrait peptidique de lupin tel que celui commercialisé par la société SILAB sous la dénomination commerciale Structurine^{®}; le beta-sitosteryl sulfate de sodium tel que celui commercialisé par la société SEPORGA sous la dénomination commerciale Phytocohésine^{®} ; et un extrait hydrosoluble de maïs tel que celui commercialisé par la société SOLABIA sous la dénomination commerciale Phytovityl^{®} ; un extrait peptidique de *Voandzeia substerranea* tel que celui commercialisé par la société Laboratoires Sérobiologiques sous la dénomination commerciale Filladyn LS 9397^{®} ; et les lignanes tels que le sécoisolaricirésinol.

### Agents anti-pelliculaires

Les agents antipelliculaires peuvent être tout agent actif utile pour prévenir l'apparition des pellicules, diminuer leur nombre et/ou les faire disparaître totalement. Ainsi, l'agent antipelliculaire peut être choisi parmi les agents antifongiques et/ou antibactériens cités ci-après.
Plus particulièrement, ces agents peuvent être choisis parmi :
- les sels de pyridinethione notamment les sels de calcium, de magnésium, de barium, de strontium, de zinc, de cadmium, d'étain et de zirconium. Le sel de zinc de pyridinethione est particulièrement préféré.
Le sel de zinc de pyridinethione est notamment commercialisé sous la dénomination Omadine de zinc par la société OLIN.
- les composés azolés tels que le climbazole, le kétoconazole, le clotrinazole, l'econazole, l'isoconazole et le miconazole.
- les polymères antifongiques tels que l'amphotéricine B ou la nystatine.
- les sulfures de sélénium en particulier ceux de formule SₓSe₈₋ₓ , x allant de 1 à 7.
- D'autres agents antipelliculaires, sont le soufre sous ses différentes formes, le sulfure de cadmium, l'allantoïne, les goudrons de houille ou de bois et leurs dérivés en particulier l'huile de cade, l'acide salicylique, l'acide undécylénique, l'acide fumarique, les allylamines telle que la terbinafine.
Il s'agit également d'agents de lutte contre les états desquamatifs du cuir chevelu qui sont choisis de préférence parmi les sels de pyridinethione comme le zinc pyrithione, les dérivés de 1-hydroxy-2-pyrrolidone comme la piroctone et la piroctone olamine ; les sulfures de sélénium comme le disulfure de sélénium ; le climbazole, l'acide undécylénique ; le Kétoconazole , le cyclopirox ou leurs mélanges. En pratique, ces agents actifs additionnels ou le mélange d'agents actifs additionnels peuvent représenter de 0,001% à 10% en poids par rapport au poids total de la composition et préférentiellement de 0,1 à 5% en poids.

### Agents anti-microbiens

Les agents antimicrobiens susceptibles d'être utilisés dans la composition selon l'invention peuvent notamment être choisis parmi le 2,4,4'-trichloro-2'-hydroxy diphényl éther (ou triclosan), le 3,4,4'-trichlorobanilide, le phénoxyéthanol, le phénoxypropanol, le phénoxyisopropanol, l'hexamidine iséthionate, le métronidazole et ses sels, le miconazole et ses sels, l'itraconazole, le terconazole, l'éconazole, le ketoconazole, le saperconazole, le fluconazole, le clotrimazole, le butoconazole, l'oxiconazole, le sulfaconazole, le sulconazole, le terbinafine, le ciclopiroxe, le ciclopiroxolamine, l'acide undécylenique et ses sels, le peroxyde de benzoyle, l'acide 3-hydroxy benzoïque, l'acide 4-hydroxy benzoïque, l'acide phytique, l'acide N-acétyl-L-cystéine, l'acide lipoïque, l'acide azélaïque et ses sels, l'acide arachidonique, le résorcinol, le 2,4,4'-trichloro-2'-hydroxy diphényl éther, le 3,4,4'-trichlorocarbanalide, l'octopirox, l'octoxyglycérine, l'octanoylglycine, le caprylyl glycol, l'acide 10-hydroxy-2-décanoïque, le dichlorophenyl imidazol dioxolan et ses dérivés décrits dans le brevet WO9318743, le farnesol, les phytosphingosines et leurs mélanges.
Les agents antimicrobiens préférés sont le triclosan, le phénoxyéthanol, l'octoxyglycérine, l'octanoylglycine, l'acide 10-hydroxy-2-décanoïque, le caprylyl glycol, le farnesol et l'acide azélaïque.

A titre d'exemple, l'agent antimicrobien peut être utilisé dans la composition selon l'invention en une quantité représentant de 0,1 à 20%, et de préférence de 0,1 à 10%, du poids total de la composition.

La présente invention concerne selon un autre de ses aspects, un procédé de traitement cosmétique pour prévenir et/ou traiter les peaux sèches, comprenant l'administration, notamment topique, d'une quantité efficace d'au moins d'un extrait de bactérie filamenteuses non photosynthétiques et non fructifiantes cultivés sur un milieu enrichi en eau de la Roche Posay, l'une de leurs fractions ou l'un de leurs métabolites.

Le procédé de traitement cosmétique de l'invention peut être mis en oeuvre notamment en appliquant les compositions telles que définies ci-dessus, selon la technique d'utilisation habituelle de ces compositions. Par exemple : applications de crèmes, de gels, de sérums, de lotions, de laits de démaquillage ou de compositions après-solaires sur la peau ou sur les cheveux secs, application d'une lotion pour cheveux sur cheveux mouillés, de shampooings, ou encore application de dentifrice sur les gencives.

Le procédé cosmétique selon l'invention peut être mis en oeuvre par administration topique. Il peut comprendre une application unique. Selon un autre mode de réalisation, l'application est répétée par exemple 2 à 3 fois quotidiennement sur une journée ou plus et généralement sur une durée prolongée d'au moins 4 semaines, voire 4 à 15 semaines, avec le cas échéant une ou plusieurs périodes d'interruption.

Dans la description et dans les exemples suivants, sauf indication contraire, les pourcentages sont des pourcentages en poids et les plages de valeurs libellées sous la forme "entre ... et ..." incluent les bornes inférieure et supérieure précisées. Les ingrédients sont mélangés, avant leur mise en forme, dans l'ordre et dans des conditions facilement déterminées par l'homme de l'art.
Les exemples ci-après sont présentés à titre illustratif et non limitatif du domaine de l'invention.

### Exemple 1 - Préparation d'un extrait bactérien selon l'invention : biomasse de Vitreoscilla filiformis cultivée sur un milieu enrichi en eau thermale de La Roche Posay

### Préparation du milieu de culture :

### Composition :

| | |
|---|---|
| * Extrait de levure | 2 à 3 g |
| * Peptone Papaïnique de soja | 2 à 3 g |
| * Glucose | 2 à 3 g |
| * Micro élément de Heller | 2 ml |
| * CaCl₂, 2H2O | 66.21 mg |
| * Eau thermale La Roche Posay | 13-14 ml. |

Cette solution mère sera diluée par de l'eau osmosée dans un rapport de 1/75 avant stérilisation.

Le pH du milieu est ajusté à 5,00 par d'une solution molaire H₃PO₄. Le milieu est stérilisé par autoclavage à 121 °C pendant 30 minutes. Après refroidissement à température ambiante, le pH est réajusté à 7,20 par rajout d'une solution molaire de KOH.

### Culture :

Après ensemencement à 1% du milieu avec la souche *Vitreoscilla filiformis* déposée à l'ATCC sous le n°15551, la culture est mise en agitation orbitale à 100 tours / min et à 26°C. Après 48 heures de croissance, la culture est centrifugée à 8000 g pendant 15 minutes. Les culots sont récupérés puis autoclavés à 121°C pendant 30 minutes. Cette biomasse peut être utilisée pour des tests d'évaluation.

### Exemple 2 - essais cliniques sur sujets atteints de dermatites atopiques

### Données cliniques :

Une première étude clinique (étude 1), en double aveugle, a évalué en intra-individuel l'effet comparatif d'une crème contenant 5% d'extrait de *Vitreoscilla filiformis* (V. f.) cultivée de façon classique (ci-après dénommé « extrait classique » sur les rougeurs survenant chez des sujets atteints de dermatites atopiques légères à modérées (lésions symétriques contre placebo).
L'extrait dit classique de *Vitreoscilla filiformis* est préparé selon les modalités suivantes :
- *Préparation du milieu de culture :*
Composition :

| | |
|---|---|
| * Extrait de levure | 2 g |
| * Peptone Papaïnique de soja | 2 g |
| * Glucose | 2 g |
| * Micro éléments de Heller | 2 ml |
| * CaCl₂, 2H2O | 66.21 mg |
| * Eau | qsp 1l |

Le pH du milieu est alors ajusté à 5,00 par d'une solution molaire H₃PO₄. Le milieu est stérilisé par autoclavage à 121 °C pendant 30 minutes. Après refroidissement à température ambiante, le pH est réajusté à 7,20 par rajout d'une solution molaire de KOH.
- *Culture :*

Au laboratoire: Après ensemencement à 1% du milieu avec la souche *Vitreoscilla filiformis,* la culture est mise en agitation orbitale à 100 tours / min et à 26°C. Après 48 heures de croissance, la culture est centrifugée à 8000 g pendant 15 minutes. Les culots sont récupérés puis autoclavés à 121°C pendant 30 minutes. Cette biomasse peut être utilisée pour des tests d'évaluation.
*- En fermenteur :* dans un fermenteur équipé préférentiellement d'un draft tube pour limiter le cisaillement, on ensemence la souche de V. f. à 1% volume minimum. Le pH est maintenu stable à 7 UpH durant toute la culture, la T° est régulée entre 26 et 28°C et l'oxygénation maintenue à 10% pO2 durant toute la culture par action soit sur la vitesse d'agitation soit par régulation du débit d'air. Ce type de culture peut être menée en batch, fed-batch ou en continu. Nous préférerons cette dernière technique qui garantit une biomasse reproductible grâce au contrôle du taux de croissance (µ). La biomasse récoltée en continu par centrifugation à 10 000 g est congelée à -20°C. Lorsque la cuve de congélation est pleine, elle est décongelée à 4°C puis conditionnée dans des emballages manipulables par un opérateur. Ces emballages contenant la biomasse sont alors stérilisés pour être stabilisés. Chaque opération de stérilisation représente alors un lot de fabrication.

Dans cette étude les produits contenant l'extrait appliqué 2 fois par jour ont été très bien tolérés.
L'extrait est formulé dans la composition 1A qui est une formule contenant 5% de l'extrait classique dans une émulsion huile dans eau/Arlacel/Myrj contenant 5% Parleam et 15% de silicone volatile. L'effet de cette composition 1A est comparé à celui d'un placébo : la composition 2A qui correspond à l'excipient : émulsion huile dans eau/Arlacel/Myrj contenant 5% Parleam et 15% de silicone volatile.

L'évolution des signes et symptômes de la dermatite atopique des patients traités en comparaison à l'effet du placebo est observé en contralatéral (p=0,008, Test de Wilcoxon). La composition 1A contenant l'« extrait classique » de *Vitreoscilla filiformis à* 5% n'a pas eu une action significative sur la sécheresse de la peau des sujets testés.

Une seconde nouvelle étude clinique (étude 2 réalisée dans les mêmes conditions que la précédente par la même équipe d'expérimentateurs) destinée à évaluer l'efficacité en intra-individuel d'une crème contenant 5% d'extrait bactérien préparé selon l'exemple 1 à montré une efficacité spécifique sur les états de sécheresse retrouvés dans les atopies légères à modérées.

L'extrait est formulé dans la composition 1 B qui est une formule contenant 5% d'extrait bactérien selon l'invention (obtenu selon l'exemple 1) dans une émulsion huile dans eau déminéralisée Arlacel/Myrj contenant 5% Parleam, 15% de cyclopentasiloxane, 3% glycérine et 2% vaseline. L'effet de cette composition 1 B est comparé à celui d'un placébo : la composition 2B qui correspond à une émulsion huile dans eau de La Roche Posay Arlacel/Myrj contenant 5% Parleam, 15% de cyclopentasiloxane, 3% glycérine et 2% vaseline.

La composition 1 B comprenant l'extrait bactérien selon l'invention est appliquée 2 fois par jour ont été très bien tolérés, la différence par rapport à l'étude ci-avant réside dans le nombre de visite et l'analyse de la rémanence de l'activité du produit.

La composition a significativement diminué l'intensité de l'excoriation et la lichénification et leur retentissement sur le prurit de la dermatite atopique des patients en comparaison à l'effet du placebo en contralatéral (p=0,0041, Test de Fisher). L'efficacité thérapeutique a été observée dans les 15 jours qui ont suivi l'application.
Cette supériorité d'efficacité par rapport à l'étude précédente est due à sa spécificité à agir sur les sècheresses de la peau observée chez ces patients. Ces extraits cultivés sur l'eau de la Roche Posay ont significativement diminué les signes et les symptômes dus à la sécheresse cutanée des patients en comparaison à l'effet du placebo en contralatéral (p=0,01, Test de Fisher).

### Exemple 3 - essais cliniques sur sujets atteints de dermites séborrhéiques

La troisième étude clinique a évalué l'efficacité d'une lotion hydroalcoolique contenant 5% d'extrait de *Vitreoscilla filiformis* préparé selon l'exemple 1 sur les dermites séborrheiques. La composition de la formule est un mélange de polyethyleneglycol stéarate, aminomethyl propanol et cyclopentadimethylsiloxan preparée dans de l'eau distillée.

Dans cette étude, les produits testés appliqués 1 fois par jour ont été très bien tolérés.

Ces produits ont significativement diminué les lésions erythémato-squameuses et le prurit de sujets présentant une dermite séborrhéique du cuir chevelu des patients en comparaison à l'effet du placebo (p<0,0001, Chi-Square). L'efficacité a été observée dans les 15 jours qui ont suivi l'application.
Il est intéressant de noter qu'il apparaît spécifiquement une diminution des lésions de desquamation (scores) de 58,6% pour la lotion hydro alcoolique contenant l'extrait bactérien selon l'invention uniquement, la lotion hydro alcoolique placebo ne faisant que peu varier les scores de desquamation entre J1 et J28 (diminution de 13,5%).
Ces analyses statistiques soulignent que le traitement par l'extrait bactérien selon l'invention permet une diminution significative du score de desquamation pendant la phase de traitement (et ceci par rapport au groupe placebo (p<0,0001))

Si on analyse la rémanence de l'activité des lotions, on remarque que la desquamation même après l'arrêt du traitement ne varie que peu à J35 par rapport à J28 (pas de différence significative entre J28 et J35 pour l'extrait bactérien selon l'invention (p=0,1698)) montrant un effet bénéfique durable dans le temps.

### Exemples 4 - Compositions topiques

### Lait pour le soin du visage des peaux sèches

| | |
|---|---|
| Chlorure de magnésium | 3,00 |
| Ascorbate de calcium | 3,00 |
| Extrait bactérien selon l'exemple 1 | 5,00 |
| Stéarate de glycérol | 1,00 |
| Alcool cétylstéarylique/alcool cétylstéarylique oxyéthyléné | |
| à 30 moles OE (Sinnowax AO^{®} vendu par la société HENKEL) | 3,00 |
| Alcool cétylique | 1,00 |
| Diméthicone (DC 200 Fluid^{®}vendu par la société | |
| DOW CORNING) | 1,00 |
| Huile de vaseline | 6,00 |
| Myristate d'isopropyle (Estol IMP 1514 vendu^{®} par UNICHEMA) | 3,00 |
| Antioxydant | 0,05 |
| Glycérine | 20,00 |
| Conservateur | 0,30 |
| Eau | qsp 100 |

### Lait pour le soin du visage des peaux sèches

| | |
|---|---|
| Ascorbate de magnésium | 3,00 |
| Huile de pépin de cassis | 4,00 |
| Huile de bourrache | 4,00 |
| Extrait bactérien selon l'exemple 1 | 5,00 |
| Stéarate de glycérol | 1,00 |
| Alcool cétylstéarylique/alcool cétylstéarylique oxyéthyléné | |
| à 3 moles OE (Sinnovax AO^{®} vendu par la société HENKEL) | 3,00 |
| Alcool cétylique | 1,00 |
| Diméthicone (DC 200 Fluid^{®} vendu par la société Dow Corning) | 1,00 |
| Huile de vaseline | 6,00 |
| Myristate d'isopropyle (Estol IPM 1514^{®} vendu par la | |
| société Unichema) | 3,00 |
| Glycérine | 20,00 |
| Conservateur | 0,30 |
| Eau | qsp 100 |

### Lotion émollient pour le corps

| | | |
|---|---|---|
| Huile minérale | | 8,0% |
| Isopropyle palmitate | | 5,0% |
| Polyglycéryl-3-diisostéarate | | 4,0% |
| Octyldodecanol | | 4,0% |
| Carbomère | | 0,3% |
| Extrait bactérien selon l'exemple 1 | | 2,0% |
| Sodium cocoyglutamate | | 2,0% |
| Hydroxyde de sodium à 10% | | 1,2% |
| Conservateur | | 0,5% |
| Parfum | | 0,5% |
| Eau | qsp | 100% |

### Shampoing antipelliculaire

| | | |
|---|---|---|
| Sodium Lauryl Sulphate | | 7,0% |
| Cocamidopropylbetain | | 2,0% |
| Sodium Lauryl sulphosuccinate | | 2,0% |
| Extrait bactérien selon l'exemple 1 | | 4,0% |
| Chlorure de sodium | | 1,0% |
| Conservateurs | | 0,5% |
| Parfum | | 0,5% |
| Eau | qsp | 100% |

### Crème pour peau sèche

| | | |
|---|---|---|
| Arachidyl behenyl alcohol/arachidylglusoside | | 3,0% |
| Isohexadecane | | 7,0% |
| Huile d'amande douce | | 3,0% |
| Beurre de Karité | | 2,0% |
| Glycérine | | 5,0% |
| Extrait bactérien selon l'exemple 1 | | 3,0% |
| BHT | | 0,05% |
| POB méthyle | | 0,1% |
| POB propyle | | 0,05% |
| Eau | qsp | 100% |

### Crème pour peaux très sèches

| | | |
|---|---|---|
| Extrait bactérien selon l'exemple 1 | | 1,5% |
| Glyceryl stearate et PEG 100 stéarate | | 5,0% |
| Isohexadecane | | 8,0% |
| Beurre de Karité | | 5,0% |
| Glycérine | | 3,0% |
| Carbopol 981 0,2% | | 0,2% |
| Lubragel | | 5,0% |
| Phenoxyéthanol | | 1,0% |
| Soude | qsp | pH6 |
| BHT | | 0,05% |
| Dc 1503 | | 1,0% |
| Eau | qsp | 100% |

## Revendications

1. Utilisation cosmétique d'au moins un extrait de bactérie filamenteuse non photosynthétique et non fructifiante cultivée sur un milieu comprenant au moins une eau thermale et/ou minérale non sulfureuse comme agent pour traiter les signes associés à la sécheresse des matières kératiniques.

2. Utilisation selon la revendication 1, **caractérisée en ce que** ladite bactérie est *Vitreoscilla filiformis.*

3. Utilisation selon la revendication 1 ou 2, **caractérisée en ce que** ladite eau est l'eau de La Roche Posay.

4. Utilisation selon l'une quelconque des revendications précédentes, **caractérisée en ce que** l'extrait est présent à raison de 0,001 à 20% en poids par rapport au poids total de la composition.

5. Utilisation selon l'une quelconque des revendications précédentes **caractérisée en ce que** ledit extrait est appliqué topiquement sur la peau.

6. Utilisation selon l'une quelconque des revendications précédente, pour traiter les états de sècheresse cutanée, les états squameux et/ou les démangeaisons et/ou tiraillements associés aux peaux sèches.

7. Utilisation selon l'une quelconque des revendications précédentes, pour traiter les désordres cutanés liés à un défaut d'excrétion et/ou de sécrétion de sébum et/ou restaurer physiologiquement un état d'hydratation convenable au *stratum corneum* et/ou traiter les peaux sèches hypo-séborrhéiques et/ou stimuler la sébogénèse.

8. Utilisation selon l'une quelconque des revendications précédentes, pour prévenir et/ou réduire les rides liées à une sécheresse cutanée.

9. Utilisation selon l'une quelconque des revendications 1 à 7, pour traiter les fibres kératiniques sèches et/ou améliorer le confort des peaux et cuirs chevelus secs.

10. Utilisation selon l'une quelconque des revendications 1 à 7, pour lutter contre l'aspect terne et/ou atone de la peau et/ou des fibres kératiniques.

11. Utilisation selon l'une quelconque des revendications 1 à 5, pour traiter les peaux sèches non pathologies constitutionnelles ou acquises.

12. Utilisation selon la revendication 11, pour traiter la peau sèche acquise induite par des paramètres extérieurs.

13. Utilisation selon la revendication 11, pour traiter la peau sèche constitutionnelle non pathologique choisie parmi la peau sénile et la xérose vulgaire.

14. Utilisation d'au moins un extrait de bactérie filamenteuse non photosynthétique et non fructifiante cultivée sur un milieu comprenant au moins une eau thermale et/ou minérale non sulfureuse pour la préparation d'une composition destinée au traitement prophylactique ou thérapeutique de la dermatite atopique, des xéroses atopiques, des dermites séborrheiques et des hyperkératoses.

15. Utilisation selon l'une quelconque des revendications précédentes, **caractérisée en ce que** ledit extrait est associé à un autre actif choisi parmi les vitamines B3, B5, B6, B8, C, E ou PP, les caroténoides, les curcuminoides et la niacine.

16. Composition comprenant au moins un extrait de bactérie filamenteuse non photosynthétique et non fructifiante cultivée sur un milieu comprenant au moins une eau thermale et/ou minérale non sulfureuse et au moins un composé choisi parmi les agents desquamants ; les agents hydratants ; les agent anti-glycation; les agents anti-inflammatoires ou apaisants ; les agents stimulant la prolifération et/ou la différenciation des kératinocytes ; les agents anti-pelliculaires ; et les agents anti-microbiens.

17. Composition selon la revendication 16, **caractérisée en ce que** ladite bactérie est *Vitreoscilla filiformis* (souche ATCC 15551).

18. Composition selon la revendication 16 ou 17, **caractérisée en ce que** ladite eau est l'eau de La Roche Posay.

19. Composition selon l'une quelconque des revendications 16 à 18, **caractérisée en ce que** l'extrait est présent à raison de 0,001 à 20% en poids par rapport au poids total de la composition.

20. Procédé de traitement non thérapeutique pour prévenir et/ou traiter les matières kératiniques sèches, les peaux sèches et/ou fragilisées, et/ou pour traiter les peaux sèches hypo-séborrhéiques, et/ou pour prévenir et/ou traiter les démangeaisons et/ou tiraillements et/ou pour stimuler la sébogénèse, et/ou pour le traitement et/ou la prévention des désordres cutanés et/ou de l'unité pilosébacée liés à un défaut d'excrétion et/ou de sécrétion de sébum, et/ou pour prévenir et/ou traiter les fibres kératiniques sèches ou fragilisées, et/ou pour prévenir et/ou réduire les rides liées à un sécheresse cutanée, comprenant l'administration d'au moins une quantité efficace d'au moins un extrait de bactérie filamenteuse non photosynthétique et non fructifiante cultivée sur un milieu comprenant au moins une eau thermale et/ou minérale non sulfureuse.

21. Procédé selon la revendication 20, **caractérisé en ce que** ladite bactérie est *Vitreoscilla filiformis* (souche ATCC 15551).

22. Procédé selon la revendication 20 ou 21, **caractérisé en ce que** ladite eau est l'eau de La Roche Posay.

23. Procédé selon l'une quelconque des revendications 20 à 22, **caractérisé en ce que** l'extrait est présent à raison de 0,001 à 20% en poids par rapport au poids total de la composition.
